(19) Europäisches Patentamt
European Patent Office
Office européen des brevets

(11) Publication number: **0 060 133**
**B1**

(12) **EUROPEAN PATENT SPECIFICATION**

(45) Date of publication of patent specification: **12.08.87**

(51) Int. Cl.$^4$: **G 01 N 33/66,**
G 01 N 33/543, A 61 K 49/00

(21) Application number: **82301188.7**

(22) Date of filing: **09.03.82**

(54) **Method and associated materials for measuring glucose level in body fluids.**

(30) Priority: **09.03.81 US 241991**
**27.01.82 US 343128**

(43) Date of publication of application:
**15.09.82 Bulletin 82/37**

(45) Publication of the grant of the patent:
**12.08.87 Bulletin 87/33**

(84) Designated Contracting States:
**AT BE CH DE FR GB IT LI LU NL SE**

(58) References cited:
**WO-A-81/00354**
**US-A-3 615 228**
**US-A-4 436 094**

**SCIENCE, vol. 206, no. 4423, December 7, 1979 Washington (US) A. CERAMI et al.: "A glucose-Controlled Insulin delivery system: semisynthetic insulin bound to lectin", pages 1190-1191**

(73) Proprietor: **EVREKA, INC.**
**16 Foster Street**
**Bergenfield New Jersey 07647 (US)**

(72) Inventor: **Cerami, Anthony**
**Flanders-Drakestown Road**
**Flanders New Jersey 07836 (US)**

(74) Representative: **Denmark, James**
**c/o Bailey Walsh & Co. 5 York Place**
**Leeds LS1 2SD Yorkshire (GB)**

(56) References cited:

**CHEMICAL ABSTRACTS, vol. 92, no. 23, June 9, 1980,page 300, abstract no. 193916j, Columbus Ohio (US) C. BORREBAECK et al.: "A binding assay of carbohydrates and glycoproteins using a lectin electrode"**

**The Merck Index, 9th. edition, page 4289**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 060 133 B1

Courier Press, Leamington Spa, England.

**Description**

The present application relates to a method and associated systems for measuring glucose in animal body fluids.

As used herein, the term "glucose" is intended to cover those blood sugars, including glucose and polysaccharides consisting of glucose subunits as are conventionally found in the blood, plasma and related body fluids. Generally, a variety of polysaccharides of differing chain length will be found, all of which are generally identified by this term, and the use of the term "glucose" is therefore intended to embrace all such sugars within its scope for the purposes of the present invention.

The detection and measurement of glucose in body fluids, such as blood, urine and cerebrospinal fluid, provides information that is crucial to a proper assessment of the functions of the body. Thus, hypo- and hyperglycemic conditions, which result from abnormal variations in blood glucose level, require prompt and accurate measurement, in instances such as the administration of emergency medical attention to patients exhibiting these conditions, to avert their deterimental and potentially fatal effects. Likewise, ongoing measurement of blood glucose levels is frequently necessary for patients with continuing diabetic conditions, to retain better control over them.

Several methods have been used in the instance of periodic measurement of glucose levels. Two methods that are currently in wide use comprise the chemical, and enzymatic method. The chemical method utilizes a sample of body fluid, that is isloated and reacted with compounds capable of oxidizing glucose and producing a color change. This technique is semi-quantitative, but has the primary drawback that it is not specific for glucose and will give false readings with other sugars and body components with which the oxidants will react.

The enzymatic method for periodic glucose measurement employs the enzyme glucose oxidase which oxidizes available glucose to form gluconic acid and hydrogen peroxide. Generally, a leuco dye is utilized, so that the hydrogen peroxide by-product may be reacted with the dye to form a particular color identifying the presence of glucose. This method is deficient in that it is a kinetic analysis that varies with temperature and time, and requires a specific apparatus with a quantitative measurement of the color bearing composition.

A known method using an enzyme is disclosed in the publication of Chemical Abstracts, Vol. 92 No. 23, June 9 1980, Page 300, Abstract No. 193916j, Columbus Ohio (US), in which there is disclosed that a sample whose glucose content is to be determined or indicated is applied to immobilised lectin together with an enzyme. The result is a complex in which the glucose is bound to the lectin in a degree proportional to the amount of glucose present, but this degree of binding can only be determined by further processing. This process is also a kinetic analysis.

The ongoing measurement of glucose levels, has been attempted by a variety of apparatuses including indwelling probes, however these devices have proved wanting for specificity and frequently suffer from interference from other biological compounds. For example, Colton et al., *Transplantation and Clinical Immunology,* Volume X, Pages 165—173, Amsterdam (1978), disclose a system including an electronic glucose sensor electrically connected to an insulin resorvoir and pump. In this system, the sensor responds to rising glucose levels, and instructs the reservior and pump to automatically dispense an appropriate quantity of insulin into the bloodstream. The sensor employs a platinum electrode catalyst for the purpose of oxidizing glucose. The use of the catalyst, however, reduces the specificity of the electrodes, as interference with other metabolites and resulting unreliability occurs.

Soeldner et al., NIH Publication No. 76—854 (1976), at Pages 267—277, propose a glucose-sensitive implant electrode, that utilizes an immobilised quantity of the enzyme glucose oxidase, that by its action on available glucose in the blood stimulates an ion exchange that causes a corresponding differential incurrent that may be sensed and reported by the electrode. The mechanism of glucose oxidase activity with body fluid, is also used in a corresponding in vitro test, where the formation of hydrogen peroxide by the reaction of the enzyme, in the presence of a leuco dye, results in a visible color reaction. The in vivo system of Soeldner et al, is deficient in that the enzyme glucose oxidase is unstable in this environment, and therefore is an unreliable determinant of glucose concentrations.

In accordance with the present invention, there is provided a method for measuring the level of glucose in animal body fluids comprising preparing a glucose indicator, which glucose indicator consist essentially of a stable, synthetically prepared reversible complex of a carbohydrate component, said carbohydrate component being a sugar selected from monosaccharides, oligosaccharides and mixtures thereof, a binding macromolecular component and a directly demonstrable indicator element associated with one of said carbohydrate component or said macromolecular component, said glucose indicator being maintained in a state of dynamic equilibrium, and being adapted to operate under thermodynamic equilibrium principles, placing a sample of said body fluid in contact with said glucose indicator and maintaining said indicator and said sample in contact with each other for a period of time sufficient for an equilibrium condition to be reached, whereby any glucose present in said body fluid displaces said carbohydrate component in said reversible complex with said macromolecular component, and observing the corresponding indicating reaction that results from the release of said

directly demonstrable indicator element and its correspondingly direct identification of both the quantitative and qualitative presence of any glucose present in said body fluid.

The macromolecular component may be carbohydrate-binding proteines such as lectins, with specific binding affinities for particular carbohydrates. In this embodiment, the indicator element is preferably a dye or a color-forming radical, that may be associated or bound to either the carbohydrate oligomer or the lectin. In operation, the reversible complex between the carbohydrate component and the lectin dissociates in contact with glucose present in the fluid sample, to the extent that such glucose is present, and this dissociation permits the indicator element to give a color reaction.

In an alternative embodiment of the invention, the glucose indicator may comprise an indwelling monitor adapted for the continuous measurement of glucose having a variable electrical charge and adapted to be disposed in registry with the system of body fluid to be monitored. The monitor is adapted to measure changes in glucose concentration as a function of changes in electrical charge, and includes a charge-transfer medium wherein the binding macromolecular component is a reversible complex, and the carbohydrate component bears an electrical charge, and thereby comprises a portion of the indicator element as well. Thus, body fluids capable of passing through the monitor and having increased glucose levels, cause the charge-bearing carbohydrate to be released to the electrical field of the monitor, with the corresponding increase in the flow of electrical charge, serving to indicate the variation in glucose level of the fluid.

The glucose monitor may comprise an electrode, with an anode and cathode spaced apart from each other, and the electrical charge-transfer medium located in between. The macromolecular and carbohydrate components, may respectively be selected from those materials specified with reference to the glucose indicator, described above. The charge-bearing carbohydrate components may comprise carbohydrates having charged substituents bound thereto, and in one embodiment, the subunits of carbohydrate oligomers may each bear a given unit charge, so that oligomers of differing chain length will offer a charge corresponding linearly in magnitude to the number of recurring carbohydrate units. Also, any incremental changes in electrical charge within the monitor, resulting from low-level inductive effect, may be easily measured by existing electrical diagnostic equipment, available for measurement of charge differentials of this order. In such way, the electrode may compensate for signal variations that are extraneous to the parameter being measured.

The present glucose indicators, may thus comprise a color-forming strip, or an electrode, each encased within a semi-permeable membrane, so that the primary components of the indicator, would remain encased, while the glucose-bearing body fluid would be capable of passing therethrough.

Specific lectins may be utilized in association with specific carbohydrates, to test for particular sugars present in the body fluid. The glucose indicator may comprise a variety or continuum of such lectin-carbohydrate oligomer complexes, each evidencing a variant indicator reaction responsive to the presence of differences in the identity or concentration of sugars present.

The glucose indicator may be prepared with one of the components irreversibly bound to a suitable and insoluble support, and enclosed within a selectively permeable membrane, and the final article may assume the form of a strip. A kit is likewise contemplated, comprising the glucose indicator in strip form and a color chart identifying the specific concentrations and identities of the sugars tested for.

The glucose monitor including the electrode described earlier, may be either implanted alone, or in conjunction with appropriate insulin dispensing means. An appropriate current-responsive meter may be selected from equipment available in the art, and specifically calibrated to reflect precise changes in glucose concentration.

The present invention offers a simple yet accurate and reliable method for determining glucose concentrations in body fluids, that provides medically significant quantitative identification of glucose. The operation of the present indicator is less subject to variations due to time and temperature parameters, and is therefore more reliable.

Accordingly, it is a principal object of the present invention to provide a method for measuring glucose levels in animal body fluids.

It is a further object of the present invention to provide a method as aforesaid, which may be utilized either as a continuous monitoring technique, or for periodic measurements.

It is a further object of the present invention to provide a method as aforesaid which yields reliable results.

It is a still further object of the present invention to provide a glucose indicator for periodic glucose level measurement, that operates in direct response to the changes in glucose concentration.

It is a still further object of the present invention to provide an indicator as aforesaid, comprising an indwelling glucose monitor adapted to measure changes in glucose concentration as a function of changes in electrical charge.

Other objects and advantages will become apparent to those skilled in the art from a consideration of the ensuing description, as well as the following drawings.

Figure 1 is a schematic plan view illustrating the spatial relation of the components of the glucose indicator of the present invention.

Figure 2 is a schematic plan view illustrating the

possible construction of a glucose monitor in accordance with the present invention.

The measurement of glucose concentrations in body fluids in accordance with the present invention, may be performed on either a periodic basis, or on a continuing basis as described hereinafter. Periodic measurement makes use of a glucose indicator which, when contacted with a sample of the body fluid, quantitatively identifies the glucose by means of the reaction of an indicator element such as a dye. In such instance, the glucose indicator comprises a reversible complex of a carbohydrate component, a binding macromolecular component and an indicator element associated with one of said components.

Continuous measurement of glucose concentrations by means of the present invention, makes use of an indwelling glucose monitor that resides in fluid registry with system of the body fluid under measurement, and measures concentration of glucose as a function of changes in electrical charge. The glucose monitor utilizes a charge-transfer medium comprising a reversible complex of a binding macromolecular component, and a charge-bearing carbohydrate component that reversibly dissociates from the macromolecular component, to cause a variation in electrical activity within the monitor that may be linearly related to the concentration of glucose in the body fluid.

An important element of both embodiments of the present invention, is the binding macromolecular component. The term "macromolecular component" as utilized herein, refers primarily to molecules that evidence reversible binding capability with other micro- or macromolecules. Examples of molecules meeting the foregoing definition include natural binding proteins, enzymes, regulatory proteins and synthetically modified binding molecules, such as chemically modified proteins. Of these, the natural proteins known as lectins are preferred herein.

Lectins are carbohydrate-binding proteins of plants and animals that exhibit a variety of specificities for carbohydrates (*Lis et al.,* Ann. Review of Biochemistry, *42,* 541 (1973); I. J. Goldstein and C. E. Hayes, Adv. In Carbohydrate Chemistry and Biochemistry, Vol. 35, R. S. Tipson and D. Horton, eds. (Academic Press, New York, 1978, pp. 128—341). Lectins, and in particular the lectin known as concanavalin A, a Jack Bean lectin, exhibit a natural affinity for various sugars which, more particularly, is a function of the number of saccharide subunits of the given sugar. For example, concanavalin A, which is specific for glucose and mannose, will not bind with galactose; particularly, specificity is shown for α-D-mannopyranose and α-D-glucopyranose.

Other lectins, such as soybean lectins show similar specificities; thus, soybean lectins are specific for β-D-N-acetylgalactosamine and α-D-galactose units, and wheat germ lectin is specific for β-D-N-acetylglucosamine. The preferred lectin, concanavalin A, is also observed to have an increased affinity for multiples of glucose up to 6.

The carbohydrate component preferably comprises a sugar, and includes the simple sugars or monosaccharides, as well as their low molecular weight condensation polymers, known as the oligosaccharides, that conventionally contain from two to nine monosaccharide units. Many of the sugars are naturally occurring, and may be found in animal body fluid. Particularly, a carbohydrate component may comprise glucose in the monomeric or oligomeric form; glucose oligomers may include other saccharides such as mannose and galactose, and may be either recovered from nature or synthetically prepared. The naturally occurring oligosaccharides are often associated with protein or lipid fractions, and may be utilized herein in such form.

The specific carbohydrate component useful in the present invention is chosen on the basis of its equivalence in affinity for the formation of the reversible complex with the macromolecular component, with the material or agent to be detected and measured by the indicators disclosed herein. Thus, for example, as the lectin concanavalin A has an increasing affinity for glucose oligomers with greater numbers of monosaccharide units; correspondingly, this affinity would extend to concentrations of glucose in a fluid sample that would correspond in range. In particular, the corresponding range of glucose concentrations in body fluids lies within the physiological range of 10—400 mg/dl.

Accordingly, a glucose indication could be prepared as illustrated in Figure 1, with a plurality of lectin molecules, each disposed on a substrate, and reversibly associated with carbohydrate components of differing size, representing a continuum of saccharide units corresponding to the physiological range of glucose. Relatively low levels of glucose concentration would be unable to displace the higher oligomers, but would readily displace the oligomers of corresponding affinity, which in turn would permit the associated indicator element to signify the presence of glucose in that concentration. The exact operation of the reversible complex is discussed below.

The reversible complex that constitutes the indicator of the embodiment of Figure 1, and the electrical charge-transfer medium of the embodiment illustrated in Figure 2, comprises a reaction between the macromolecular component and the carbohydrate component, as generally noted above. This reaction must be reversible and non-covalent. The bonding that occurs between the respective components is caused by non-covalent forces such as hydrophobic and ionic hydrogen bonding forces. Such interactions are known in the art, and their effects on molecular affinity and recognition have been described, for example, in *Korolkovas et al.*, "Essentials of Medicinal Chemistry", pp. 44—81, John Wiley & Sons, 1976, and the particular reactions of proteins and carbohydrates has been reviewed in Goldstein, I. J. ed., *Carbohydrate-Protein*

*Interaction*, ACS Symposium Series No. 88 (1979). An example of a reversible interaction is the interaction between an enzyme and its substrate or a competitive inhibitor thereof.

As described earlier in brief, the present reversible complex between the carbohydrate component and the macromolecular component operates in a state of dynamic equilibrium, as the material in the body fluid being tested, for, and the carbohydrate component compete for association with the macromolecular component. In the instance where the macromolecular component is a lectin, and the material under test is glucose at certain levels of concentration, the glucose indicator illustrated in Figure 1, and the charge-transfer medium of the glucose monitor illustrated in Figure 2, both participate in an equilibrium that arises between the glucose at the particular concentration and the reversible complex bearing a corresponding carbohydrate component, to complex with the lectin.

In the instance of the embodiment of Figure 1, the displacement of the carbohydrate component of a particular reversible complex permits the indicator associated with that complex, to signify the presence of the particular concentration of glucose, preferably by a color-forming reaction. In the instance where the glucose indicator comprises an indwelling monitor, the displacement of the electrical charge-bearing carbohydrate component of a particular reversible complex permits the carbohydrate to effect the level of electrical charge in the monitor, to thereby signify the presence of the particular concentration of glucose.

In the instance where a glucose indicator is prepared for the periodic measurement of glucose levels in body fluid, the indicator element may be associated with either the carbohydrate component or the macromolecular component, for the sole function of signifying the dissociation of these components, and the corresponding presence of glucose. In this embodiment, therefore, the indicator component may be a color-forming material such as a dye or a color-forming radical that is freed or otherwise chemically altered by the dissociation of the carbohydrate and the macromolecular components, to produce a color-forming reaction. The indicator element may also include a compound or portion thereof that forms a precipitate upon such dissociation, and the invention therefore is not limited to color-forming materials exclusively.

For example, a dye such as an arylhydrazine may be reacted with the reducing end of a carbohydrate oligomer to form a stable addition product such as an arylosazone, which will give a color reaction when the carbohydrate component and the macromolecular component are dissociated. By utilizing different hydrazines in combination with different oligomers responsive to individual concentrations of glucose, it is possible to develop a mixture of ligands exhibiting variations in color formation and affinity for particular lectins, whereby different concentrations of glucose will cause the formation of differing color reactions. Referring to Figure 1, the disposition of different dyes with different chain length oligomers illustrates the manner in which the discrete levels of glucose concentration in fluid samples can be identified.

The indicator element of the first embodiment of the invention, may be associated with the macromolecular component, rather than the carbohydrate component, however, with the same manner of operation. Thus, the specific dyes such as rhodamine, isothiocyanate and 4 - dimethylaminoazobenzene - 4′ - sulfinyl chloride, that are capable of reacting with proteins, may be incorporated onto the macromolecular components. In this embodiment, the carbohydrate component, rather than the macromolecular component would be fixedly attached to a substrate and the dissociation of the carbohydrate and the macromolecular component would set the latter free, permitting the attached dye to form its color reaction. The disposition of the respective components on the substrate will be discussed later on with reference to the preparation of the indicator of the present invention.

In a further embodiment, the indicator element may comprise a particular functional group that may be associated with the carbohydrate or the macromolecular component, and which would be capable of forming the colored complex with an immobilized dye included with the glucose indicator, when the respective component bearing the functional group is displaced. For example, a sulfhydryl group may be incorporated into the carbohydrate component, and would, upon its release, react with a number of reagents, such as 5,5′-dithiobis (2-nitrobenzoic acid), to develop a color.

In general, the method of the present invention comprises contacting the indicators described herein with a quantity of an animal body fluid, and maintaining the indicators in contact with the fluid for a period of time sufficient for the indicators to signify the presence and quantity, if any, of the material being tested for, in this case glucose. Thus, analysis of body fluid for the presence of glucose may be conducted by placing the fluid in contact with the glucose indicators, and maintaining such contact for a period of time sufficient to enable the reversible complex between a particular macromolecular component and its corresponding carbohydrate component, to dissociate in favor of a complex involving the macromolecular component and the glucose, which latter event will be signified by the indicator. In the instance, for example, where the indicator comprises a lectin such as Concanavalin A reversibly bound to a glucose oligomer bearing a particular dye, the indicator would be placed in contact with a drop of body fluid which would be maintained in contact with the indicator for a period of time, such as, for example, up to five minutes, whereupon equilibrium between the glucose present and the reversible complex will

have been reached, and the indicator element comprising the dye will have given its color reaction.

In the instance where the glucose indicator comprises an indwelling monitor or electrode, the monitor may be either temporarily or permanently implanted in the body in registry with the system of body fluid to be measured. The monitor is then electrically connected to appropriate current generating and current measuring means. The glucose in the body fluid passing through the monitor competes for the immobilized lectin, and results in the release of the charge-bearing carbohydrate to permit the latter to change the electrical charge of the monitor. The reversible nature of the complex permits the charge-bearing carbohydrate to reassociate with the lectin, in the instance where the glucose level in the body fluid drops.

The glucose indicator of the embodiment illustrated in Figure 1, and the charge-transfer medium of the glucose monitor, illustrated in Figure 2, may both be prepared as follows. The particular lectin or lectins chosen for use may be fixed to a suitable insoluble support, such as cellulose, agarose, plastic and glass, by either covalent bonding or non-covalent adsorption. The technique of solid state immobilization of enzymes and other proteins on resins, films, test tubes, and glass beads are well known (see e.g. Zaborsky, C. *"Immobilized Enzymes"*, CRC Press, Cleveland, 1973; Lowe, C. R. and Dean, P. D. G., "The Chemistry of Affinity Chromatography", *Affinity Chromatography*, John Wiley and Sons, N.Y., 1974; Axen, et al., U.S. Patent No. 3,645,852; and Kraemer, et al., U.S. Patent No. 4,039,413). For example, the lectin may be attached to a cellulose support by activation of the support with cyanogen halide, reaction with cyanuric acid, periodate oxidation, epoxide formation, and reaction with various bifunctional reagents, such as bis-oxirane, dimethyl adipimate, phenol-2,4-disulfonyl chloride, and divinyl sulphone. Preferably, a cellulose strip is reacted with cyanogen bromide, and the thus activated cellulose strip is then incubated with concanavalin A, and the reaction later stopped by the addition thereto of glycine.

With respect to the indicator illustrated in Figure 1, it is to be understood that this embodiment includes the instance where the carbohydrate component is anchored to a substrate by techniques equally well known in the art, so that the various mono- and oligosaccharides may be permanently bound to the substrate and reversibly associated with the respective lectins.

As noted earlier, the specific carbohydrate components may be either recovered from nature or synthetically prepared. Particular carbohydrates may be prepared by, for example, limited acid hydrolysis of mannin to form oligomers of varying length which are then separable by chromatography, to recover the specific oligosaccharides.

In the instance where the in vitro indicator of Figure 1 is being prepared, the carbohydrate components recovered above may either be fixedly bound to the substrate, or reacted with various dyes to form the conjugates illustrated schematically in Figure 1. Thus, oligomers of varying chain length may bear different dyes as illustrated, and as described in detail earlier.

In the instance where the charge-transfer medium of the embodiment of Figure 2 is under preparation, the recovered carbohydrate components may be treated to dispose one or more charges thereon. In particular, ionic charges may be disposed on the carbohydrates by reacting appropriate substituents or moieties with either the hydroxyl groups of the carbohydrates, or the aldehyde group disposed at the reducing end of the carbohydrate. A variety of substituents having negative charges could be so introduced, as follows:

$$—SO_3^{\ominus};\ —CH_2—C(=O)—O^{\ominus}$$

or

$$—CH_2—CH_2—\overset{\oplus}{N}H_2—CH_2CH_3$$

exhibiting a positive charge and arylhydrazines having electrically charged substituents. Naturally, the foregoing substituents are merely illustrative of those materials that might be reacted with the carbohydrate components to acheive the ionic or charged state, as the invention encompasses all equivalent substituents that are non-toxic to the body fluid within its scope.

Once both of the components are prepared, the carbohydrate and macromolecular components are brought into contact with each other, and establish the reversible complexes described earlier with respect to each of the embodiments of the present invention. Thus, for example, the glucose indicator of Figure 1 may be prepared in strip form, and, after the reversible complexes are formed, may be covered with a semi-permeable membrane designed to permit glucose and the dye-carbohydrate complex to diffuse readily therethrough. In such instance, a variety of semi-permeable membranes are well known and may be used for in vitro testing equipment of this type, and include several natural and synthetic resinous materials evidencing pore size, conducive to this application.

Referring again to Figure 1, the schematically illustrated strip would be placed in contact with the fluid sample, with the pores of the membrane sufficiently large to permit the passage of glucose therethrough, to compete for the formation of a reversible complex with the support-bound lectin. Upon the displacement of the carbohydrate component by the glucose, the dye, such as "Dye 1", would be free to form a color reaction to signify its displacement, to indicate the concentration of the glucose in the investigated body fluid.

The indicator described with reference to Figure 1, could be prepared as part of a kit, including the indicator in strip form, together with a suitable color chart providing a spectrum of colors, and their quantitative or qualitative significance, to facilitate the rapid analysis of test results. Thus, a series of colors would be outlined that would denote particular glucose concentration levels to enable the user of the kit to rapidly determine glucose levels from the color reaction of the indicator strip. The simplicity of this kit, would facilitate its use by both trained medical staff, as well as the patients themselves.

The preparation and use of the glucose monitor comprising the alternate embodiment of the present invention, may be understood with reference to Figure 2. Thus, a monitor is schematically illustrated, comprising electrode 10, having a housing 12 with an anode 14 and a cathode 16 mounted inside, and in spaced apart relation to each other. As the present illustration is schematic, it can be visualized that conventional means for external electrical connection can be provided to interface with anode 14 and cathode 16, to provide means for current circulation and measurement in conjunction with an appropriate source of electric current, and current metering means, not shown, which may be connected to electrode 10 in electrical series therewith.

In accordance with conventional electrode construction, a source of electrical current transfer is disposed within housing 12, to permit the passage of charge between anode 14 and cathode 16. In the present electrode, this charge source may comprise in whole or in part, electrical charge-transfer medium 18, which, as schematically illustrated, comprises a segment of immobilized macromolecular material, such as a lectin, that is either bound to a solid substrate, or is itself polymerized and solidified. The lectin 20 may have attached thereto one or more charge-bearing carbohydrates such as illustrated at 22. Carbohydrate 22 is shown with recurring saccharide units that bear negatively charged substituents 24. Substituents 24 may comprise negatively charged moieties that may be chemically attached to the respective carbohydrate by reaction either with one or more of the hydroxyl groups of the carbohydrate, or by attachment of the moiety to the aldehyde at the reducing end thereof. Also, the charge of a given carbohydrate subunit may vary from that of other subunits, to give further differentiation and specificity to particular glucose fractions in the body fluid. In this way, further refinement of the electrical charge-transfer medium is possible, to define both quantitatively and qualitatively, the presence of particular sugars in body fluids. The preparation of the charge-bearing carbohydrate components in this fashion, is described earlier herein.

In similar fashion to the preparation of the indicator of Figure 1, the charge-transfer medium 18 may be prepared with a continuum of charge-bearing carbohydrates 22, each carbohydrate having a differing subunit number and a correspondingly different magnitude of charge. Likewise, carbohydrates of differing composition, i.e. variant saccharides, may be bound to the same lectin 20, to provide a wide spectrum of glucose detection.

Referring again to Figure 2, housing 12 provides a means for the passage therethrough of the body fluid, to permit the ingress and egress of glucose, schematically illustrated and labelled 26. To this end, a membrane 28 may be provided, surrounding all or a portion of housing 12, to provide selective permeability facilitating the free movement therethrough of glucose.

A variety of polymeric materials may be utilized to prepare membrane 28, and such materials may exhibit selectivity based upon differentials in porosity, as well as surface charge. In such instance, the polymeric materials may form into the membrane, bearing the desired surface charge, or the formed membrane may subsequently be treated to provide such charge thereon.

Suitable polymeric materials may be selected from positively charged and negatively charged materials, as well as materials possessing both positive and negative charge. For example, positively charged materials may comprise polyvinyl pyridine; negatively charged materials may be selected from polyacrylic acid and polyethylene terepthalate; and a polymeric material possessing a combined positive and negative charge may comprise a polystyrene sulfonate-vinylbenzyl trimethyl ammonium chloride copolymer. Naturally, the foregoing materials are merely illustrative of suitable polymers that may be utilized in preparation of membrane 28, and the invention is accordingly not limited to these specific materials, but rather encompasses those materials possessing the requisite porosity and charge capability set forth above.

As noted earlier, charge-transfer medium 18, including lectin 20 and charge-bearing carbohydrates 22 may be prepared generally in the same manner outlined with respect to the embodiment of Figure 1. Thus, after the individual lectins 20 are fixed to a suitable insoluble support, and the particular charge-bearing carbohydrates 22 are prepared, these respective components may be brought in contact with each other to establish the desired reversible complexes of medium 18. After the formation of these reversible complexes, medium 18 may be mounted within housing 12, between anode 14 and cathode 16 as illustrated. Housing 12 may then be covered with membrane 28 to complete the assembly of monitor 10.

The operation of monitor 10 for continuously measuring glucose levels of an in vivo basis comprises the implantation of the monitor within the body of the animal, and in registry with the system of body fluids to be observed. As mentioned earlier, the monitor may remain

implanted either temporarily or indefinitely, and may be electrically connected to current generating and measuring means, to provide a calibrated continuous indication of glucose levels. As noted earlier, the current measuring means or gauge may be appropriately calibrated, so that specific incremental variations in glucose level may be noted and appropriate remedial action taken. In such connection, the present invention may be utilized in combination with an appropriate dispensing means, for the automatic administration of an antidote such as insulin, in the instance where the present invention is applied to a diabetic condition by fluid registry with the bloodstream.

As the present monitor will be required to operate in fluid media containing a variety of ions other than those that exist or may be formed by the action of the present charge-transfer medium, one or more reference electrodes may be appropriately linked to the monitor for the purpose of detecting ions that are unrelated to the function of the monitor. For example, such electrodes may sense the presence of inorganic ions having no relation to the level of glucose, and could provide correction to the present monitor to avoid false indication of increased glucose levels. Linkage of these reference electrodes is well known, and may be accomplished by connection in a Wheatstone Bridge arrangement or equivalent disposition, to permit the reference electrodes to provide the needed correction. The particular form of such connection does not form a part of the present invention.

**Claims**

1. A method for measuring the level of glucose in animal body fluids comprising preparing a glucose indicator, which glucose indicator consists essentially of a stable, synthetically prepared reversible complex of a carbohydrate component, said carbohydrate component being a sugar selected from monosaccharides, oligosaccharides and mixtures thereof, a binding macromolecular component and a directly demonstrable indicator element associated with one of said carbohydrate component or said macromolecular component, said glucose indicator being maintained in a state of dynamic equilibrium, and adapted to operate under thermodynamic equilibrium principles, placing a sample of said body fluid in contact with said glucouse indicator and maintaining said indicator and said sample in contact with each other for a period of time sufficient for an equilibrium condition to be reached, whereby any glucose present in said body fluid displaces said carbohydrate component in said reversible complex with said macromolecular component, and observing the corresponding indicating reaction that results from the release of said directly demonstrable indicator element and its corresponding direct identification of both the quantitative and qualitative presence of any glucose present in said body fluid.

2. The method of Claim 1 wherein said binding macromolecular component comprises a binding protein, and said indicator element is selected from direct color-forming materials capable of forming precipitates, and mixtures thereof.

3. The method of Claim 1, wherein said glucose monitor includes a charge-transfer medium comprising a reversible complex of binding macromolecular component, and a charge-bearing carbohydrate component, said reversible complex being adapted to react with said glucose to change the level of electrical charge sensed by said glucose monitor, said glucose monitor being placed in fluid registry with the body fluid of said animal, for which glucose level measurement is to be made, said monitor being maintained in fliud registry with said body fluid for a period of time sufficient for said body fluid to permeate said monitor, and for any glucose present in said body fluid to react with said reversible complex, and said glucose level being measured by measuring the level of electrical charge sensed by said monitor, said level of electrical charge varying as a function of the concentration of said glucose.

4. The method of Claim 1, wherein said glucose monitor comprises an electrode having an anode, a cathode spaced apart thereform, and said electrical charge-transfer medium disposed therebetween.

5. The method of Claim 1 or 4, wherein said binding macromolecular component comprises a binding protein, said charge bearing carbohydrate component comprises a sugar having an ionic charge disposed thereon, said sugar being selected from monosaccharides, oligosaccharides, and mixtures thereof.

6. A glucose indicator for use in measuring the level of glucose in animal body fluids comprising a reversible complex of carbohydrate component, a binding macromolecular component, and an indicator element associated with one of said components, said reversible complex being adapted to dissociate in the presence of glucose in said body fluid, whereby said glucose forms a complex with said macromolecular component, and said indicator element is released and signifies the presence of said glucose.

7. The indicator of Claim 6, wherein said carbohydrate component comprises a sugar selected from monosaccharides, oligosaccharides, and mixtures thereof, said binding macromolecular component being selected from the group consisting of natural binding proteins, synthetic binding proteins and mixtures thereof, and said indicator element being selected from color-forming materials, precipitate forming materials and mixtures thereof.

8. A glucose monitor for the continuous, in vivo measurement of glucose in animal body fluids comprising:

A. an electrode adapted to electrically sense

variations in the concentration of said glucose in said body fluid:

B. said electrode including a charge-transfer medium comprising a reversible complex of a binding macromolecular component, and a charge-bearing carbohydrate component:

C. said reversible complex being adapted to react with said glucose to change the level of electrical charge sensed by said electrode, to thereby indicate corresponding variations in said glucose concentration.

9. The monitor of Claim 8, wherein said electrode comprises:

A. an electrode housing:

B. an anode and a cathode mounted in said housing and spaced apart from each other: and

C. said charge-transfer material being disposed between said anode and said cathode.

10. The monitor of Claim 8, wherein said charge-bearing carbohydrate component comprises a sugar having one or more reactive sites thereof bound to an ionic substituent, said sugar being selected from monosaccharides, oligosaccharides, and mixtures thereof.

11. The monitor of Claim 9 wherein said ionic substituent is selected from the group consisting of

$$-SO_3^-;\quad -CH_2-\overset{\overset{\large O}{\|}}{C}-O^{\ominus};$$

$$-CH_2CH_2-\overset{\oplus}{N}H_2-CH_2CH_3;$$

arylhydrazines having electrically charged substituents and mixtures thereof.

12. A method of the continuous in vitro measurement of glucose in animal body fluids comprising:

A. preparing a glucose monitor adapted to electrically sense variations in the concentration of said glucose in said body fluid, said glucose monitor including a charge-transfer medium comprising a reversible complex of a binding macromolecular component, and a charge-bearing carbohydrate component, said reversible complex being adapted to react with said glucose to change the level of electrical charge sensed by said glucose monitor;

B. placing said glucose monitor in fluid registry with the body fluid to be monitored;

C. maintaining said glucose monitor in fluid registry with said body fluid for a period of time sufficient for said body fluid to premeate said glucose monitor, and for any glucose present in said body fluid to react with said reversible complex; and

D. measuring the level of electrical charge sensed by said glucose monitor;

wherein said level of electrical charge varies as a function of the concentration of said glucose, and said monitor is adapted to continually measure variations therein.

13. The method of Claim 12 wherein said charge-bearing carbohydrates are retained within said glucose monitor, to enable said glucose monitor to sense reductions in said glucose concentration, and to perform said continual measurement.

14. The method of Claim 12 or 13 wherein said glucose concentration if measured with reference to a current-responsive meter calibrated for such purpose.

15. The method of Claim 12 wherein said glucose monitor comprises an electrode having an anode, a cathode spaced apart therefrom, and said electrical charge-transfer medium disposed therebetween.

16. The method of Claim 12 or 15 wherein said charge-bearing carbohydrate component is retained within said monitor by a selectively permeable membrane.

17. The method of Claim 12 or 15, wherein said binding macromolecular component comprises a binding protein, said charge-bearing carbohydrate component comprises a sugar having an ionic charge disposed thereon, said sugar being selected from monosaccharides, oligosaccharides, and mixtures thereof.

18. The method of Claim 17 wherein said binding macromolecular component is fixedly attached to an inert, insoluble substance, and said charge-bearing carbohydrate component is bound to said macromolecuar component in a reversible complex.

19. The method of Claim 12 wherein said charge-bearing carbohydrate component has bound thereto, at least one ionic substituent.

20. The method of Claim 15 wherein said charge-bearing carbohydrate component has bound thereto, at least one ionic substituent.

**Patentansprüche**

1. Ein Verfahren zum Messen des Glukosespiegels in tierischen Körperflüssigkeiten mit Herstellen eines Glukoseindikators, der im wesentlichen aus einem stabilen, synthetisch zubereiteten reversiblen Komplex einer Kohlehydratkomponente besteht, die ein Zucker aus Monosacchariden, Oligosacchariden und Mischungen aus diesen ist, und einer bindenden großmolekularen Komponente und einem direkt nachweisbaren Indikatorelement, das mit der Kohlehydrat- oder der großmolekularen Komponente eng zusammengehört, wobei der Glukoseindikator im Zustand eines dynamischen Gleichgewichtes gehalten wird und unter thermodynamischen Gleichgewichtsbedingungen wirken kann, wobei eine Probe der Körperflüssigkeit mit dem Glukoseindikator in Berührung gebracht und der indikator und die Probe während einer zum Erreichen eines Gleichgewichtszustandes ausreichenden Zeit miteinander in Berührung gehalten werden, wodurch jegliche in der Körperflüssigkeit enthaltene Glukose die Kohlehydratkomponente in dem reversiblen Komplex mit der großmolekularen Komponente verdrängt, und die

entsprechende Anzeigreaktion, die sich aus der Freisetzung des direkt nachweisbaren Indikatorelementes und seiner entsprechenden direkten Identifizierung von sowohl der quantitativen als auch der qualitativen Gegenwart jeder in der Körperflüssigkeit enthaltenen Glukose ergibt, beobachtet wird.

2. Das Verfahren nach Anspruch 1, wobei die bindende großmolekulare Komponente ein bindendes Protein ist und das Indikatorelement aus direkten farbbildenden Stoffen, die Niederschläge und Mischungen aus diesen bilden können, ausgewählt wird.

3. Das Verfahren nach Anspruch 1, wobei der Glukosemonitor ein ladungsübertragendes Medium aus einem reversiblen Komplex einer bindenden großmolekularen Komponente und einer ladungstragenden Kohlehydratkomponente enthält, und der reversible Komplex mit der Glukose unter Änderung der von dem Glukosemonitor erfaßten Stärke der elektrischen Ladung reagiert, der Glukosemonitor mit der Köperflüssigkeit des Tieres, für das die Glukosespiegelmessung durchzuführen ist, in Berührung gebracht wird, der Monitor mit der Körperflüssigkeit während eines Zeitabschnittes in Berührung gehalten wird, der ausreicht, damit die Körperflüssigkeit den Monitor durchdringt, und jegliche in der Körperflüssigkeit enthaltene Glukose mit dem reversiblen Komplex reagiert, und der Glukosespiegel durch Messen der Stärke der von dem Monitor erfaßten elektrischen Ladung gemessen wird und die Stärke der elektrischen Ladung als eine Funktion der Konzentration der Glukose schwankt.

4. Das Verfahren nach Anspruch 1, wobei der Glukosemonitor eine Elektrode mit einer Anode und einer von dieser abgelegenen Kathode enthält und das die elektrische Ladung übertragende Medium dazwischen angeordnet ist.

5. Das Verfahren nach Anspruch 1 oder 4, wobei die bindende großmolekulare Komponente ein bindendes Protein ist und die ladungstragende Kohlehydratkomponente ein Zucker mit einer auf ihm angeordneten Ionenladung ist und der Zucker aus Monosacchariden, Oligosacchariden und Mischungen aus diesen ausgewählt ist.

6. Ein Glukoseindikator zur Verwendung beim Messen des Glukosespiegels in tierischen Körperflüssigkeiten mit einem reversiblen Komplex einer Kohlehydratkomponente, einer bindenden großmolekularen Komponente und einem mit einer dieser Komponenten eng zusammengehörenden Indikatorelement, wobei der reversible Komplex in der Gegenwart von Glukose in der Körperflüssigkeit dissoziiert, durch die Glukose mit der großmolekularen Komponente einen Komplex bildet und das Indikatorelement freigesetzt wird und die Gegenwart von Glukose anzeigt.

7. Der Indikator nach Anspruch 6, wobei die Kohlehydratkomponente ein aus Monosacchariden, Oligosacchariden und Mischungen aus diesen ausgewählter Zucker ist, die bindende großmolekulare Komponente aus der Gruppe bestehend aus natürlichen bindenden Proteinen, synthetischen bindenden Proteinen und Mischungen aus diesen und das Indikatorelement aus farbbildenden Stoffen, Ausfälle bildenden Stoffen und Mischungen aus diesen ausgewählt ist.

8. Ein Glukosemonitor für die Kontinuierliche in-vivo-Messung von Glukose in tierischen Körperflüssigkeiten aus:

A. einer Elektrode zum elektrischen Erfassen von Schwankungen in der Konzentration der Glukose in der Körperflüssigkeit:

B. wobei diese Elektrode ein eine Ladung übertragendes Medium aus einem reversiblen Komplex einer bindenden großmolekularen Komponente und einer eine Ladung tragenden Kohlehydratkomponente ist:

C. wobei der reversible Komplex mit der Glukose unter Änderung des von der Elektrode erfaßten Spiegels der elektrischen Ladung reagiert, um damit entsprechende Schwankungen in der Glukosekonzentration anzuzeigen.

9. Der Monitor nach Anspruch 8, wobei die Elektrode besteht aus:

A. einem Elektrodengehäuse,

B. einer in dem Gehäuse befestigten Anode und Kathode, die einen Abstand voneinander aufweisen, und

C. wobei das die Ladung übertragende Material zwischen der Anode und der Kathode angeordnet ist.

10. Der Monitor nach Anspruch 8, wobei die die Ladung übertragende Kohlehydratkomponente ein Zucker ist, von dem ein oder mehrere reaktive Stellen an ein ionisches Substitut gebunden sind und der Zucker aus Monosacchariden, Oligosacchariden und Mischungen aus diesen ausgewählt ist.

11. Der Monitor nach Anspruch 9, wobei das ionische Substitut aus der Gruppe aus

$$-SO_3^-;\quad -CH_2-\overset{\overset{\displaystyle O}{\|}}{C}-O^{\ominus};$$

$$-CH_2CH_2-\overset{\oplus}{N}H_2-CH_2CH_3;$$

Arylhydrazinen mit elektrisch geladenen Substituten und Mischungen aus diesen ausgewählt ist.

12. Ein Verfahren für die kontinuierliche invitro-Messung von Glukose in tierischen Körperflüssigkeiten aus:

A. Zubereiten eines Glukosemonitors zum elektrischen Erfassen von Schwankungen in de Konzentration der Glukose in der Körperflüssigkeit, wobei der Glukosemonitor ein eine Ladung übertragendes Medium aus einem reversiblen Komplex einer bindenden großmolekularen Komponente und eine eine Ladung tragende Kohlehydratkomponente enthält, und der reversible Komplex mit der Glukose unter Änderung des von dem Glukosemonitor erfaßten Spiegels der elektrischen Ladung reagieren kann;

B. Verbringen des Glukosemonitors in Berührung mit der zu überwachenden Körperflüssigkeit;

C. Beibehalten der Berührung des Glukosemonitors mit der Körperflüssigkeit für einen Zeitabschnitt, der ausreicht, damit die Körperflüssigkeit den Glukosemonitor durchdringt und damit jegliche in der Körperflüssigkeit vorhandene Glukose mit dem reversiblen Komplex reagieren kann, und

D. Messen des Spiegels der von dem Glukosemonitor erfaßten elektrischen Ladung;

wobei der Spiegel der elektrischen Ladung als eine Funktion der Konzentration der Glukose schwankt und der Monitor die in der Glukose auftretenden Schwankungen kontinuierlich messen kann.

13. Das Verfahren nach Anspruch 12, wobei die die Ladung tragenden Kohlehydrate in dem Glukosemonitor zurückgehalten werden, damit der Glukosemonitor Verminderungen in der Glukosekonzentration erfaßt und die kontinuierliche Messung durchführen kann.

14. Das Verfahren nach Anspruch 12 oder 13, wobei die Glukosekonzentration unter Bezug auf ein für diesen Zweck geeichtes stromempfindliches Meßgerät gemessen wird.

15. Das Verfahren nach Anspruch 12, wobei der Glukosemonitor eine Elektrode mit einer Anode und von dieser einen Abstand aufweisenden Kathode aufweist und das die elektrische Ladung übertragende Medium zwischen diesen angeordnet ist.

16. Das Verfahren nach Anspruch 12 oder 15, wobei die die Ladung tragende Kohlehydratkomponente in dem Monitor durch eine selektive permeable Membrane zurückgehalten wird.

17. Das Verfahren nach Anspruch 12 oder 15, wobei die bindende großmolekulare Komponente ein bindendes Protein un die die Ladung tragende Kohlehydratkomponente ein Zucker mit einer auf ihm angeordneten ionischen Ladung ist und der Zucker aus Monosacchariden, Oligosacchariden und Mischungen aus diesen ausgewählt ist.

18. Das Verfahren nach Anspruch 17, wobei die bindende großmolekulare Komponente an einer inerten unlöslichen Substanz fest angebracht ist und die die Ladung tragende Kohlehydratkomponente in einem reversiblen Komplex an die großmolekulare Komponente gebunden ist.

19. Das Verfahren nach Anspruch 12, wobei die die Ladung tragende Kohlehydratkomponente mindestens ein an sie gebundenes ionisches Substitut aufweist.

20. Das Verfahren nach Anspruch 15, wobei die die Ladung tragende Kohlenhydratkomponente mindestens ein an sie gebundenes ionisches Substitut aufweist.

**Revendications**

1. Procédé pour mesurer le taux de glucose dans des fluides corporels d'un animal comprenant les étapes consistant à préparer un indicateur de glucose, lequel indicateur de glucose est constitué essentiellement d'un complexe stable réversible préparé par synthèse d'un composant de carbohydrate, ledit composant de carbohydrate étant un sucre choisi parmi les monosaccharides, les oligosaccharides et leurs mélanges, un composant macromoléculaire de liaison et un élément indicateur directement démontrable associé audit composant de carbohydrate ou audit composant macromoléculaire, ledit indicateur de glucose étant maintenu dans un état d'équilibre dynamique, et étant adapté pour fonctionner selon des principes d'équilibre thermodynamique, placer un échantillon dudit fluide corporel en contact avec ledit indicateur de glucose et maintenir ledit indicateur et ledit échantillon en contact l'un avec l'autre pendant une période de temps suffisante pour qu'une condition d'équilibre soit atteinte, par quoi tout glucose présent dans ledit fluide corporel déplace ledit composant de carbohydrate dans ledit complexe réversible avec ledit composant macromoléculaire, et observer la réaction indicatrice correspondante qui résulte de la libération dudit élément indicateur directement démontrable et l'indication directe de façon correspondante d'à la fois la présence qualitative et la présence quantitative de tout glucose présent dans ledit fluide corporel.

2. Procédé selon la revendication 1, dans lequel ledit composant macromoléculaire de liaison comprend une portéine de liaison, et ledit éément indicateur est choisi parmi des matériaux de formation de couleur directs capables de former des précipités, et leurs mélanges.

3. Procédé selon la revendication 1, dans lequel ledit détecteur de glucose comporte un milieu de transfert de charge comprenant un complexe réversible d'un composant macromoléculaire de liaison et d'un composant de carbohydrate porteur de charge, ledit complexe réversible étant adapté pour réagir avec ledit glucose pour modifier le niveau de charge électrique détecté par ledit détecteur de glucose, ledit détecteur de glucose étant placé en registre de fluide avec le fluide corporel dudit animal, pour lequel la mesure du taux de glucose doit être réalisée, ledit détecteur étant maintenu en registre de fluide avec ledit fluide corporel pendant une période de temps suffisante pour que ledit fluide corporel passe à travers ledit détecteur, et pour que tout glucose présent dans ledit fluide corporel réagisse avec ledit complexe réversible, et ledit taux de glucose étant mesuré en mesurant le niveau de charge électrique par ledit détecteur, ledit niveau de charge électrique variant en fonction de la concentration du glucose.

4. Procédé selon la revendication 1, dans lequel ledit détecteur de glucose comprend une électrode ayant une anode, une cathode espacée de celle-ci, et ledit milieu de transfert de charge électrique disposé entre elles.

5. Procédé selon la revendication 1 ou la revendication 4, dans lequel ledit composant macromoléculaire de liaison comprend une protéine de liaison, ledit composant de

carbohydrate porteur de charge comprenant un sucre ayant une charge ionique placée sur celui-ci, ledit sucre étant choisi parmi des monosaccharides, des oligosaccharides, et leurs mélanges.

6. Indicateur de glucose utilisable pour mesurer le taux de glucose dans des fluides corporels d'un animal, comprenant un complexe réversible d'un composant de carbohydrate, un composant macromoléculaire de liaison, et un élément indicateur associé avec l'un desdits composants, ledit complexe réversible étant adapté pour se dissocier en présence de glucose dans ledit fluide corporel, par quoi ledit glucose forme un complexe avec ledit composant macromoléculaire, et ledit élément indicateur est libéré et indique la présence du glucose.

7. Indicateur selon la revendication 6, dans lequel ledit composant de carbohydrate comprend un sucre choisi parmi des monosaccharides, des oligosaccharides, et leurs mélanges, ledit composant macromoléculaire de liaison étant choisi dans le groupe constitué de protéines de liaison naturelles, de protéines de liaison de synthèse et leurs mélanges, et ledit élément indicatuer étant choisi parmi des matériaux de formation de couleur, des matériaux formant des précipités, et leurs mélanges.

8. Détecteur de glucose pour la mesure continue in vivo du glucose dans des fluides corporels d'un animal comprenant:

A. une électrode adaptée pour détecter électriquement des variations de la concentration du glucose dans ledit fluide corporel;

B. Ladite électrode comportant un milieu de transfert de charge comprenant un complexe réversible d'un composant macromoléculaire de liaison et d'un composant de carbohydrate porteur de charge;

C. Ledit complexe réversible étant adapté pour réagir avec le glucose pour modifier le niveau de charge électrique détecté par ladite électrode, pour ainsi indiquer des variations correspondantes de la concentration du glucose.

9. Détecteur selon la revendication 8, dans lequel ladite électrode comprend:

A. Un boîtier d'électrode;

B. une anode et une cathode montées dans ledit boîtier en étant espacées l'une de l'autre; et

C. ladite matière de transfert de charge disposée entre ladite anode et ladite cathode.

10. Détecteur selon la revendication 8, dans lequel ledit composant de carbohydrate porteur de charge comprend un sucre ayant un ou plusieurs sites réactifs liés à un substituant ionique, ledit sucre étant choisi parmi des monosaccharides, des oligosaccharides, et leurs mélanges.

11. Détecteur selon la revendication 10, dans lequel ledit substituant ionique est choisi dans le groupe constitué de

$$—SO_3^-;\quad —CH_2—\overset{\overset{\displaystyle O}{\|}}{C}—O^{\ominus};$$

$$—CH_2—CH_2—\overset{\oplus}{N}H_2—CH_2CH_3,$$

des arylhydrazines ayant des substituants chargés électriquement, et leurs mélanges.

12. Procédé pour la mesure continue in vitro du glucose dans des fluides corporels d'un animal comprenant les étapes consistant à:

A. préparer un détecteur de glucose adapté pour détecter électriquement des variations de concentration du glucose dans ledit fluide corporel, ledit détecteur de glucose comportant un milieu de transfert de charge comprenant un complexe réversible d'un composant macromoléculaire de liaison et d'un composant de carbohydrate porteur de charge, ledit complexe réversible étant adapté pour réagir avec le glucose pour modifier le niveau de charge électrique détecté par ledit détecteur de glucose;

B. placer ledit détecteur de glucose en registre de fluide avec le fluide corporel à contrôler;

C. maintenir ledit détecteur de glucose en registre de fluide avec ledit fluide corporel pendant une période de temps suffisante pour que ledit fluide corporel passe au travers du détecteur de glucose, et pour que tout glucose présent dans ledit fluide corporel réagisse avec ledit complexe réversible; et

D. mesurer le niveau de charge électrique détecté par ledit détecteur de glucose;

dans lequel le niveau de charge électrique varie en fonction de la concentration de glucose, et ledit détecteur est adapté pour mesurer de façon continue des variations de celui-ci.

13. Procédé selon la revendication 12, dans lequel lesdits carbohydrates porteurs de charge sont retenus à l'intérieur du détecteur de glucose, pour permettre au détecteur de glucose de détecter des réductions de la concentration de glucose, et réaliser ladite mesure continue.

14. Procéde selon la revendication 12 ou la revendication 13, dans lequel la concentration de glucose est mesurée en référence à un appareil de mesure sensible au courant, calibré dans ce but.

15. Procédé selon la revendication 12, dans lequel ledit détecteur de glucose comprend une électrode ayant une anode et une cathode espacées l'une de l'autre, et ledit milieu de transfert de charge électrique disposé entre elles.

16. Procédé selon la revendication 12, ou la revendication 15, dans lequel ledit composant de carbohydrate porteur de charge est retenu à l'intérieur dudit détecteur par une membrane sélectivement perméable.

17. Procédé selon la revendication 12 ou la revendication 15, dans lequel ledit composant macromoléculaire de liaison comprend une protéine de liaison, ledit composant de carbohydrate porteur de charge comprend un sucre ayant une charge ionique disposée sur celui-ci, ledit sucre étant choisi parmi des monosaccharides, des oligosaccharides, et leurs mélanges.

18. Procédé selon la revendication 17, dans lequel ledit composant macromoléculaire de

liaison est fixé à une substance inerte insoluble, et ledit composant de carbohydrate porteur de charge est lié audit composant macromoléculaire dans un complexe réversible.

19. Procédé selon la revendication 12, dans lequel ledit composant de carbohydrate porteur de charge est lié à au moins un substituant ionique.

20. Procédé selon la revendication 15, dans lequel ledit composant de carbohydrate porteur de charge est lié à au moins un substituant ionique.

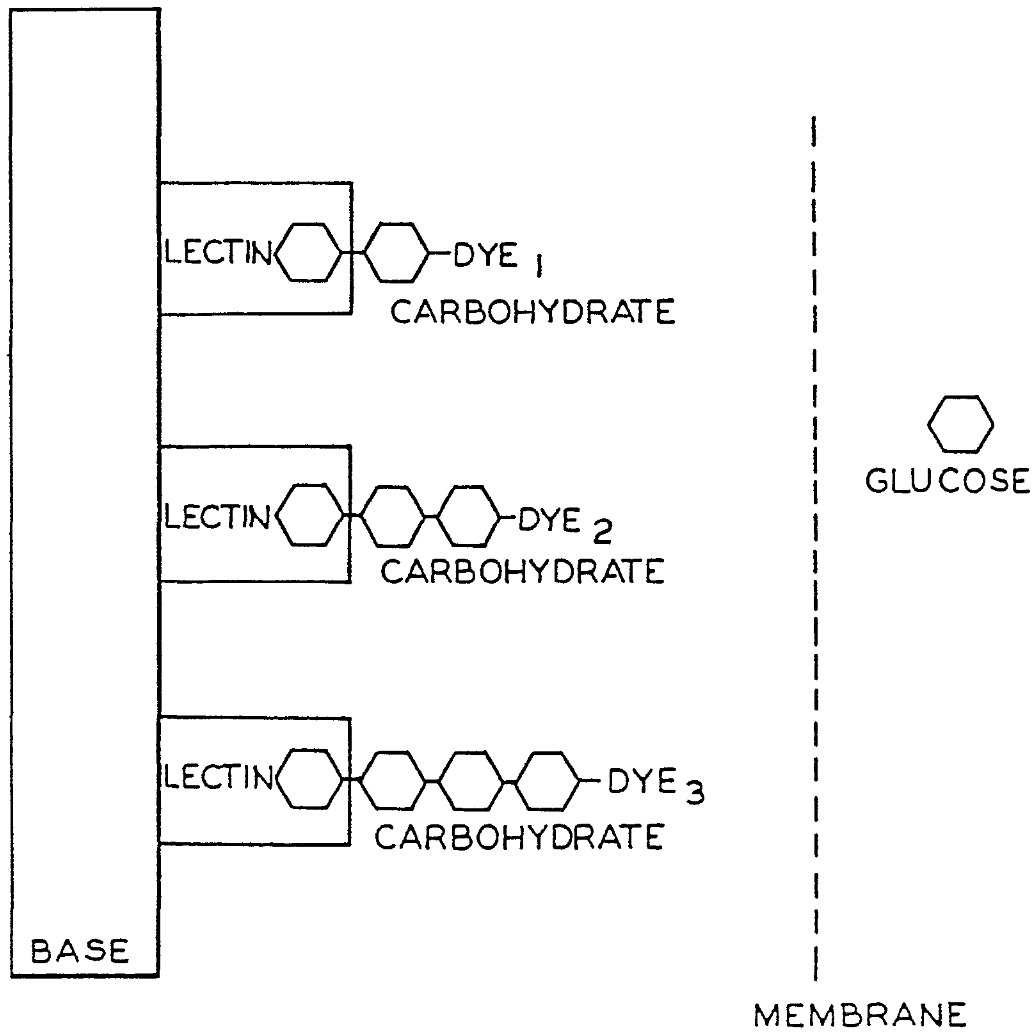
LECTIN
DYE 1
CARBOHYDRATE
LECTIN
DYE 2
CARBOHYDRATE
LECTIN
DYE 3
CARBOHYDRATE
BASE
GLUCOSE
MEMBRANE

FIG. I

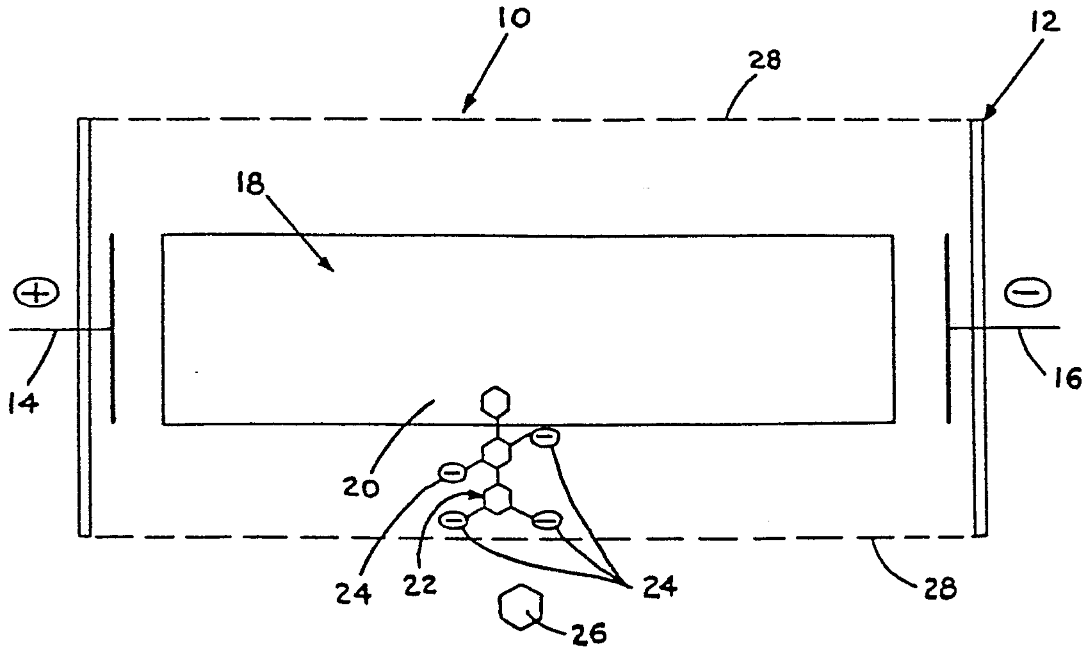


FIG. 2